# EUROPEAN PATENT APPLICATION

(11) **EP 3 608 673 A1**
(43) Date of publication of application: **12.02.2020**
(21) Application number: 18200907.6
(22) Date of filing: 17.10.2018
(51) Int. Cl.: G01N 33/68, G01N 33/74

(54) **PRO-ADM FOR PROGNOSING THE RISK OF A MEDICAL CONDITION REQUIRING HOSPITALIZATION IN PATIENTS WITH SYMPTOMS OF INFECTIOUS DISEASE**

(30) Priority: 08.08.2018 EP 18188098
(71) Applicant: B.R.A.H.M.S GmbH, 16761 Hennigsdorf (DE)
(72) Inventor: WILSON, Darius, 79539 Lörrach (DE); Bermejo Martín, Jesus Francisco, 42002 Soria (ES); Garcia Ortiz, Luis, 37003 Salamanca (ES); Herrero Rodriguez, Carmen, 37005 Salamanca (ES)
(74) Representative: Hertin und Partner Rechts- und Patentanwälte PartG mbB

(57) **Abstract**

The invention relates to a method for therapy guidance, stratification and/or control in a patient with symptoms of an infectious disease, comprising a prognosis of disease progression, the method comprising: providing a sample from said patient, and determining a level of proADM or fragment(s) thereof in said sample, wherein a low risk level of proADM level or fragment(s) thereof in said sample indicates that the patient is not at risk of a disease progression to a condition that requires hospitalization, wherein said low risk level is preferably equal or below 1.2 nmol/I ± 20%, and/or wherein a high risk level of proADM level or fragment(s) thereof in said sample indicates that the patient is at risk of a disease progression to a condition that requires hospitalization, wherein said high risk level is preferably above 1.2 nmol/I ± 20%. The invention further relates to a test kit for carrying out the method.

## Description

The invention relates to a method for therapy guidance, stratification and/or control in a patient with symptoms of an infectious disease, comprising a prognosis of disease progression, the method comprising: providing a sample from said patient, and determining a level of proADM or fragment(s) thereof in said sample, wherein a low risk level of proADM level or fragment(s) thereof in said sample indicates that the patient is not at risk of a disease progression to a condition that requires hospitalization, wherein said low risk level is preferably equal or below 1.2 nmol/l ± 20%, and/or wherein a high risk level of proADM level or fragment(s) thereof in said sample indicates that the patient is at risk of a disease progression to a condition that requires hospitalization, wherein said high risk level is preferably above 1.2 nmol/l ± 20%. The invention further relates to a test kit for carrying out the method.

### BACKGROUND OF THE INVENTION

Many patients present to their general practitioner (GP) with an infection and are sent home either with or without oral antibiotics. However, a minority of these patients can deteriorate rapidly if the underlying infection is more significant. This phenomenon is observed in the popular media regularly, i.e a patient visits their GP with mild symptoms, are sent home, and subsequently develop a potentially fatal infection. After worsening of their symptoms, these patients then go to the emergency department (ED) and are harder to treat due to the progression of the infection.

Accordingly, many GPs err on the side of caution and send too many patients to hospital based on clinical signs and symptoms alone. This causes an unnecessary strain on the hospital since the vast majority of these patients don't actually need to be hospitalised. One key feactor in decreasing ED admissions is to actually stop patients presenting to the ED in the first place, which includes those referred by their GP, if the infection is not severe.

Accordingly, a need exists in the field of appropriately treating patients with symptoms of an infectious disease, especially those presenting to a general practitioner with relatively mild symptoms. A need is therefore evident for the therapy guidance, stratification and/or control in patients with such symptoms, in particular with respect to the timing of treatment, and accurately assessing when and to which level of intensity treatment should be initiated, for example whether hospitalization is necessary. Additionally, a need exists for means to reduce unnecessary hospitalizations based on initial but non-threatening symptoms.

Employing proADM represents such means, in particular mid-regional proadrenomedullin (MR-proADM).

The data disclosed herein demonstrates that proADM enables effective prognostic statements regarding whether hospitalization is required, thereby providing effective means for therapy guidance in a patient with symptoms of infectious disease.

### SUMMARY OF THE INVENTION

In light of the difficulties in the prior art, the technical problem underlying the present invention is the provision of means for therapy guidance in a patient with symptoms of an infectious disease. In particular, one technical problem underlying the present invention is the provision of means for determining, based on simple assay to be carried out by primary health care providers, such as a general medical practitioner, whether hospitalization is necessary for patients with symptoms of infectious diease.

The present invention therefore relates to methods, kits and further means for diagnosis, prognosis, prediction, risk assessment and/or risk stratification, or therapy guidance, stratification and/or control of a patient with symptoms of infectious disease on the basis of adrenomedullin (ADM) levels, in particular proADM or MR-proADM, determined in a sample from a patient.

One object of the invention is therefore the use of a biomarker, or combination of biomarkers, to distinguish patients who are more likely or have a high risk of requiring hospitalization (ie high risk of requiring intensive treatments provided in hospital) from patients who have a low risk of requiring such treatment.

The solution to the technical problem of the invention is provided in the independent claims. Preferred embodiments of the invention are provided in the dependent claims.

The invention therefore relates to a method for therapy guidance, stratification and/or control in a patient with symptoms of an infectious disease, comprising a prognosis of disease progression, the method comprising:
- providing a sample from said patient, and
- determining a level of proADM or fragment(s) thereof in said sample,
- wherein a low risk level of proADM level or fragment(s) thereof in said sample indicates that the patient is not at risk of a disease progression to a condition that requires hospitalization, wherein said low risk level is equal or below 1.2 nmol/l ± 20%, and/or
- wherein a high risk level of proADM level or fragment(s) thereof in said sample indicates that the patient is at risk of a disease progression to a condition that requires hospitalization, wherein said high risk level is above 1.2 nmol/l ± 20%.

It represents a surprising finding that a cut-off value of proADM of or above 1.2 nmol/l ± 20% (preferably using an immunoassay fpr MR-proADM) enables a reliable indication that a patient is likely to experience worsening of their medical condition, such that hospitalization is necessary. Studies such as described below, based on proADM assessments of primary health care providers and comparisons to the frequency of hospital admission, have not been previously described in the art. As such, the prognostic statements described herein appear to represent a surprising and beneficial finding in the field of infectious disease prognosis and patient managment in a primary care and(or hospital setting.

The term "indicates that the patient is, or is not, at risk of disease progression to a condition that requires hospitalization" relates in some embodiments to the prognosis of a worsening of patient condition. In some embodiments, this term relates to a prognosis of whether certain treatments, which are only or primarily available in hospital, are necessary. In some embodiments, this term relates to prognosis of disease outcome. Although proADM is known to predict disease outcome for some medical conditions, the present invention presents particular cut-off values applicable in primary care scenarios, in particular patient groups, which would not have been derived from the prior art or suggested previously in the context of predicting potential hospitalization.

In some embodiments, treatments commonly received in hospital relate to treatments excluding topical and/or oral antibiotic administration. Examples of treatments commonly received in hospital relate further to intravenous administration of antibiotics, or other fluid therapy, such as fluid management, fluid resuscitation, fluid replacement and/or fluid redistribution, such as of colloids and/or crystalloids, or blood transfusions. Other treatments primarily available in hospital are, but are not limited to, renal replacement therapy (RRT), artificial and mechanical ventilation, surgery or cleaning procedures, such as focus cleaning.

The term "hospitalization" refers preferably, in some embodiments, to hospital admission. This differs from being referred to hospital, being assessed, and subsequently released (sent home). Hospital admission refers preferably to maintained control and/or assessment of the medical condition of a patient in the hospital, whether it be in an emergency room, ward, intensive care station, or other area of a hospital or clinic. Admission preferably, in some embodiments, relate to overnight admission of the patient for monitoring.

In one embodiment, the level of proADM or fragment(s) thereof is determined in a sample obtained by the first medical personnel to consult with the patient after occurrence of the symptoms of infectious disease.

In embodiments of the invention the term "first medical personnel to consult with the patient after occurrence of the symptoms of infectious disease" relates to, but is not limited to, general practitioners (GP), home health care workers or care givers, nurses in residences for retirees, ambulance drivers, or workers, paramedics, doctors making home visits, nurses in community clinics, and the like. The first medical personnel is preferably construed to encompass and medical staff, whether a doctor or other care giver, who first encounters a patient who has developed symptoms of infectious disease, but who has preferably not been diagnosed with a sever illness.

It is entirely surprising that based on a single proADM measurement an accurate and reliable conclusion can be made as to whether the patient will go on to develop a disease of such severity that hospitalization is required. This prognostic ability of proADM is, to the knowledge of the inventors, novel and surprising.

In one embodiment, the patient is presented in a primary care unit and a high risk level of proADM or fragment(s) thereof indicates that hospitalization of the patient is required, and wherein a low risk level of proADM or fragment(s) indicates that hospitalization of the patient is not required.

In preferred embodiments of the present invention the method is defined by the prognosis of potential hospitalization in a patient group, for whom it was previously difficult, if not impossible, to determine by routine clinical and/or molecular diagnostic means, whether a serious risk existed of serious deterioration of medical conditions, thereby requiring hospitalization. This patient group may be considered patients with mild symptoms of an infectious disease, or patients with no symptoms of serious infectious disease, e.g. without symptoms of sepsis.

In one embodiment, at the time of sample provision the patient shows mild symptoms of an infectious disease corresponding to a qSOFA score of 0 or 1.

In one embodiment, at the time of sample provision the patient shows no clinical symptoms for a blood stream infection, a sepsis, a severe sepsis and/or septic shock in the patient.

In one embodiment, the level of proADM or fragment(s) thereof in said sample in between 0.7 nm/L ± 20% and 1.2 nmol/L ± 20% indicates the presence of the disease and that the patient is not at risk of a disease progression to a condition that requires a hospitalization.

The range of proADM values from 0.7 nm/L ± 20% to 1.2 nmol/L ± 20% is of particular benefit, as healthy patients often have proADM levels from 0.0 to 0.7 nmol/L. In some embodiments, patients with over 1.5 nmol/L also require serious treatment. Therefore, there previously existed a "gray zone" of uncertainty for medical professionals, when ADM values from 0.7 nm/L ± 20% to 1.2 nmol/L ± 20% were measured. The present invention however now enables reliable prognostic statement for such patients. In some embodiments, these patients are ruled out from hospitalization, as they do not exhibit a significant risk of progressing to a serious condition requiring hospitalization.

In one embodiment, a high risk level of proADM or fragments thereof indicates that the patient is at risk of disease progression to a life-threatening condition within 48 hours, 24 hours, preferably 12 hours and wherein a low risk level of proADM indicates that the patient is not at risk of disease progression to a life-threating condition within 48 hours, 24 hours, preferably 12 hours.

In one embodiment, the level of proADM or fragment(s) thereof in said sample is indicative of the risk of a development of a blood stream infection, sepsis, severe sepsis and/or septic shock in the patient that requires hospitalization.

In one embodiment, a high risk level of proADM thereof indicates that the patient is at risk to develop a blood stream infection, sepsis, severe sepsis and/or septic shock that requires hospitalization within 48 hours, 24 hours, preferably 12 hours and wherein a low risk level of proADM indicates that the patient is not at risk to develop blood stream infection, a sepsis, a severe sepsis and/or a septic shock within 48 hours, 24 hours, preferably 12 hours.

In one embodiment, the level of proADM or fragment(s) thereof in said sample is indicative of the patient requiring frequent monitoring and/or critical care treatment. In one embodiment, the patient with a high risk level will require a medical treatment provided in a hospital setting. Examples of these treatments include but are not limited to fluid therapy, intravenous antibiotics, in some embodiments essentially any treatment beyond oral antibiotics, which may be self-administered at home.

In one embodiment, the method additionally comprises:
- determining at least one clinical score, preferably the NEWS score, for the patient suspected of having an infectious disease,
- wherein the at least one clinical score and the level of proADM or fragment(s) thereof is indicative of the presence of the infectious disease and whether the patient is at risk of a disease progression to a condition that requires a hospitalization.

In one embodiment, the infectious disease is a respiratory infectious disease, urinary tract infectious disease, a skin infectious disease or an abdominal infectious disease.

In one embodiment, determining a level of proADM or fragment(s) thereof comprises determining a level of MR-proADM in the sample. As demonstrated in the examples below, MR-proADM, preferably determined using established immunoassay products of B.R.A.H.M.S (Hennigsdorf, Germany), shows a reliable and effective prognosis according to the methods described herein. Alternative ADM molecules, including precursors or fragments, may also be employed.

In one embodiment, the sample is selected from the group consisting of a blood sample, such as a whole blood sample, a serum sample or a plasma sample, and/or a urine sample.

In one embodiment, the sample is isolated from the patients within 24 hours, 6 hours, 2 hours, 1 hour, more preferably within 30 minutes or 15 minutes after the symptoms have been determined.

In one embodiment, the method additionally comprises:
a. determining a level of at least one additional biomarker or fragment(s) thereof in a sample from said patient, wherein the at least one additional biomarker preferably is PCT or fragment(s) thereof, and
b. wherein the level of the at least one additional biomarker is indicative of an initiation of treatment with an anti-infective agent, preferably an antibiotic or antimitotic agent.

In one embodiment, at least one additional biomarker is PCT or fragment(s) thereof and a level of PCT or fragment(s) thereof above 0,25 ng/ml, preferably above 0.5 ng/ml is indicative of an initiation of a treatment with an anti-infective agent, preferably an antibiotic or antimitotic agent.

As demonstratetd in more detail below, the combined measurement of additional biomarkers, in particular PCT, enables improved prognosis and/or further indications on the extent, severity or type of infection tha is present in the subject. The combined measurement of PCT and proADM may, in some embodiments, lead to the decision regarding hospitalization in addition to the antibiotic dose and means of administration to the patient. The combination of these biomarkers therefore provides a beneficial and - in the particular patient group and practitioner group of the present invention - surpring effect.

A further aspect of the invention relates to a kit for carrying out the method as described herein.

In one embodiment, the kit comprises:
a. detection reagents for determining the level of proADM or fragment(s) thereof in a sample from a patient, and
b. reference data for the risk of a patient as to whether a disease progression to a condition that requires hospitalization will occur, in particular reference data for a risk threshold or cut-off value, wherein said reference data is preferably stored on a computer readable medium and/or employed in the form of computer executable code configured for comparing the determined levels of proADM or fragment(s) thereof with the risk threshold or cut-off value,
c. and optionally, detection reagents for determining the level of at least one additional biomarker or fragment(s) thereof, preferably PCT, in a sample from a patient, and reference data, such as reference levels for said at least one additional biomarker, preferably PCT, for a risk threshold or cut-off value, wherein said reference data is preferably stored on a computer readable medium and/or employed in the form of computer executable code configured for comparing the determined levels of the at least one additional biomarker or fragment(s) thereof with the threshold or cut-off value.

In some embodiments, the features of the kit may be considered to include those features used in characterizing the method of the invention, and vice versa.

The invention further relates to a method for therapy guidance, stratification and/or control in a patient with symptoms of an infectious disease, comprising a prognosis of disease progression, the method comprising:
- providing a sample from said patient,
- determining a level of proADM or fragment(s) thereof in said sample, and
- comparing the level of proADM or fragment(s) thereof in said sample to a cut-off value,
- wherein said cut-off value is 1.2 nmol/l ± 20%,
- wherein a (low risk) level of proADM or fragment(s) thereof in said sample below said cut-off value indicates that the patient is not at risk of a disease progression to a condition that requires hospitalization, and wherein a high risk level of proADM or fragment(s) thereof in said sample above said cut-off value indicates that the patient is at risk of a disease progression to a condition that requires hospitalization.

The method therefore relates to a method of treating an infectious disease in a patient, including electing the patient for treatment employing a method for therapy guidance, stratification and/or control in a patient with symptoms of an infectious disease, comprising a prognosis of disease progression, the method comprising:
- providing a sample from said patient,
- determining a level of proADM or fragment(s) thereof in said sample, and
- comparing the level of proADM or fragment(s) thereof in said sample to a cut-off value,
- wherein said cut-off value is 1.2 nmol/l ± 20%,
- wherein a (low risk) level of proADM or fragment(s) thereof in said sample below said cut-off value indicates that the patient is not at risk of a disease progression to a condition that requires hospitalization, and wherein a high risk level of proADM or fragment(s) thereof in said sample above said cut-off value indicates that the patient is at risk of a disease progression to a condition that requires hospitalization.

The method of treatment preferably includes the administration of suitable therapeutic measures to said patient using preferably one or more of the particular therapies described herein.

In one embodiment, the invention relates to or comprises a method for treating a patient with symptoms of an infectious disease, the method comprising the steps of:
- determining whether the patient has a high or low risk level of proADM, by:
- obtaining or having obtained a biological sample from the patient;
- performing or having performed an assay that determines a level of proADM or fragment(s) thereof in said sample;
- wherein a low risk level of proADM level or fragment(s) thereof in said sample is equal or below 1.2 nmol/l ± 20%, and/or
- wherein a high risk level of proADM level or fragment(s) thereof in said sample is above 1.2 nmol/l ± 20%,
- wherein when a high risk level of proADM level or fragment(s) thereof in said sample is determined, the patient is treated, said treatment preferably including hospitalization, or a medical treatment available only or primarily at a hospital.

The present invention therefore provides a new, quick and reliable test for primary healthcare practitioners, such as doctors, nurses, etc., to quickly assess the risk of patient with symptoms of infectious disease to develop a condition nrequiring hospitalization, such as sepsis.

The cut-off values disclosed herein refer preferably to measurements of the protein level of proADM or fragments thereof in a blood sample, preferably a whole blood sample or plasma or serum sample obtained from a patient, by means of the Thermo Scientific BRAHMS KRYPTOR assay. Accordingly, the values disclosed herein may vary to some extent depending on the detection/measurement method employed, and the specific values disclosed herein are intended to also read on the corresponding values determined by other methods.

In embodiments of the invention, the cut-off value of proADM or fragment(s) thereof that may define either a low or a high severity level, may be any value around 1.2 nmol/L ± 20%. In other embodiments, the cut-off value of proADM or fragment(s) thereof that may define either a low or a high severity level, may be any value around 1.2 nmol/L ± preferably 15%, or 12%, or 10%, or 9, 8, 7, 6, 5, 4, 3, 2, or 1%.

Any value within this range (ie within the window of variation of the 1.2 nmol/L value) may be considered an appropriate cut-off value between high and low proADM severity levels. Furthermore, values below such a cut-off value may be indicative of the absence of significant risk of developing a condition requiring hospitalization, and values equal or above such a cut-off value may be indicative of developing a condition requiring hospitalization.

Appropriate cut-off levels that may be used in the context of the present invention, comprise, without limitation, any value between 0.96 and 1.44 nmol/L. Other values include 0.95, 1.0, 1.05, 1.1, 1.15, 1.2, 1.25, 1.3, 1.35, 1.4, and 1.45 nmol/L, or a value in between these values, or range using these values as an end point.

In embodiments of the invention, deviations from these possible cut-off values are also claimed, such as deviations of ± 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1%.

In the embodiments described herein, the severity levels are defined preferably by one or more cut-off values, that represent a boundary between low and high severity levels. Any embodiments that present cut-offs therefore may use the format of a single cut-off value as a boundary between two severity levels, or a single cutoff level for each severity level.

In certain embodiments of the method of the invention, the condition requiring hospitalization is an infection-related complication, such as an infection, a nosocomial infection, sepsis and/or septic shock. In certain embodiments of the method of the invention, the condition comprises an infection, a nosocomial infection, sepsis and/or septic shock, preferably based on a respiratory infectious disease, urinary tract infectious disease, a skin infectious disease or an abdominal infectious disease.

It is particular advantage that the cut-off of proADM or fragment(s) thereof to be used in the context of the method of the invention can be used to rule-out hospitalization. Accordingly, the method enables a more accurate assessment of the prognosis/risk of a patient, depending on the situation of sample isolation and the further information available at that time, for example an increased risk of developing severe condition.

Depending on the result of the method of the present invention, embodiments of the method may comprise subsequent therapeutic decisions and/or therapeutic actions. Such therapeutic decisions may include the initiation, change or modification of medical treatment. Preferably, if the method of the present invention is indicative of development to a condition requiring treatment in hospital, suitable therapeutic measures, such as initiation or change of a certain medication, surgery or fluid therapy may be initiated.

Any therapy, medical treatment or therapeutic action disclosed herein can be employed in the context of the method of the invention as a subsequent therapeutic decision or therapeutic action, in particular if the therapeutic measure is specifically administered in hospital, including but not limited to intravenous fluid therapy, dialysis, management of electrolyte abnormalities (in particular potassium, calcium and phosphorus). Furthermore, a maintained intensive observation and care of the patient may be indicated potentially over extended periods of time, such as several day, weeks or even months. This may involve keeping or moving the patient to an ICU and/or prolonging the stay of the patient in an ICU.

On the other hand, if the result of the method of the present invention is indicative of the absence of risk of developing a condition requiring hospitalization, no specific treatment measures with respect to such complication may be required, or less serious, self-administratable treatments may be prescribed.

In one embodiment the invention additionally comprises informing the patient of the results of the diagnostic method described herein. In embodiments of the invention, the patient is at least 18 years old.

In embodiments of the invention, the sample is selected from the group consisting of a blood sample, a serum sample, a plasma sample and/or a urine sample. Determining proADM or fragment(s) thereof in blood, serum or plasma is particularly advantageous since it has been shown to be particularly accurate. Furthermore, these samples reflect the actual status of the patient at a given time-point very accurately.

In embodiments of the invention, determining a level of proADM or fragment(s) thereof comprises determining a level of MR-proADM in the sample. The employment of determining MR-proADM is preferred for any given embodiment described herein and may be considered in the context of each embodiment, accordingly. In preferred embodiments the "ADM fragment" may be considered to be MR-proADM.

According to certain embodiments, the method of the invention additionally comprises
- determining a level of at least one additional biomarker or fragment(s) thereof in a sample from said patient, wherein the at least one additional biomarker preferably is PCT or fragment(s) thereof and/or lactate, and/or
- determining at least one clinical score, wherein the at least one clinical score is preferably SOFA,
- wherein the level of the at least one additional biomarker and/or the at least one clinical score, and the level of proADM or fragment(s) thereof is indicative of whether hospitalization is required, or if the patient is at risk of developing a condition requiring treatment in hospital.

Further additional markers that may be determined in the context of the present invention comprise lactate and CRP. Clinical scores that may be determined in the context of the present invention comprise SI score (systemic inflammation score), SOFA, qSOFA, SIRS, SAPS II, APACHE II, preferably SOFA.

Determining proADM or fragment(s) thereof, and preferablySOFA, in the context of the method of the invention turned out to provide further accuracy with respect to prognosis of a subsequent condition requiring hospital admission.

In further embodiments, the at least one additional biomarker is at least one marker of lactate, rhabdomyolysis, such as creatine kinase (CK), lactate dehydrogenase (LDH), creatinine, myoglobin, aldolase, troponin, carbonic anhydrase type 3, fatty acid-binding protein (FABP), transaminases or potassium.

The detection reagents for determining the level of proADM or fragment(s) thereof, and optionally for determining the level of PCT, lactate and/or C-reactive protein or fragment(s) thereof, are preferably selected from those necessary to perform the method, for example antibodies directed to ADM, suitable labels, such as fluorescent labels, preferably two separate fluorescent labels suitable for application in the KRYPTOR assay, sample collection tubes.

The embodiments and features disclosed in the context of the method of the invention also apply to the kit of the present invention and vice versa.

In one embodiment of the method described herein the level of proADM or fragment(s) thereof and optionally additionally other biomarkers such as for example PCT or fragment(s) thereof is determined using a method selected from the group consisting of mass spectrometry (MS), luminescence immunoassay (LIA), radioimmunoassay (RIA), chemiluminescence- and fluorescence- immunoassays, enzyme immunoassay (EIA), Enzyme-linked immunoassays (ELISA), luminescence-based bead arrays, magnetic beads based arrays, protein microarray assays, rapid test formats such as for instance immunochromatographic strip tests, rare cryptate assay, and automated systems/analyzers.

The method according to the present invention can furthermore be embodied as a homogeneous method, wherein the sandwich complexes formed by the antibody/antibodies and the marker, e.g., the proADM or a fragment thereof, which is to be detected remains suspended in the liquid phase. In this case it is preferred, that when two antibodies are used, both antibodies are labelled with parts of a detection system, which leads to generation of a signal or triggering of a signal if both antibodies are integrated into a single sandwich.

Such techniques are to be embodied in particular as fluorescence enhancing or fluorescence quenching detection methods. A particularly preferred aspect relates to the use of detection reagents which are to be used pair-wise, such as for example the ones which are described in US 4 882 733 A, EP-B1 0 180 492 or EP-B1 0 539 477 and the prior art cited therein. In this way, measurements in which only reaction products comprising both labelling components in a single immune-complex directly in the reaction mixture are detected, become possible.

For example, such technologies are offered under the brand names TRACE® (Time Resolved Amplified Cryptate Emission) or KRYPTOR®, implementing the teachings of the above-cited applications. Therefore, in particular preferred aspects, a diagnostic device is used to carry out the herein provided method. For example, the level of the proADM protein or a fragment thereof, and/or the level of any further marker of the herein provided method are determined. In particular preferred aspects, the diagnostic device is KRYPTOR®.

In one embodiment of the method described herein the method is an immunoassay and wherein the assay is performed in homogeneous phase or in heterogeneous phase.

In further embodiments of the method described herein, the method additionally comprises a molecular analysis of a sample from said patient for detecting an infection. The sample used for the molecular analysis for detecting an infection preferably is a blood sample. In a preferred embodiment the molecular analysis is a method aiming to detect one or more biomolecules derived from a pathogen. Said one or more biomolecule may be a nucleic acid, protein, sugar, carbohydrades, lipid and or a combination thereof such as glycosylated protein, preferably a nucleic acid. Said biomolecule preferably is specific for one or more pathogen(s). According to preferred embodiments, such biomolecules are detected by one or more methods for analysis of biomolecules selected from the group comprising nucleic acid amplification methods such as PCR, qPCR, RT-PCR, qRT-PCR or isothermal amplification, mass spectrometry, detection of enzymatic activity and immunoassay based detection methods. Further methods of molecular analysis are known to the person skilled in the art and are comprised by the method of the present invention.

In one embodiment of the method described herein a first antibody and a second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to said proADM or fragments thereof to be detected, a measurable signal which permits detection of the resulting sandwich complexes in the measuring solution is generated.

In one embodiment of the method described herein the labelling system comprises a rare earth cryptate or chelate in combination with a fluorescent or chemiluminescent dye, in particular of the cyanine type.

In one embodiment of the method described herein, the method additionally comprises comparing the determined level of proADM or fragment(s) thereof to a reference level, threshold value and/or a population average corresponding to proADM or fragments thereof in patients who have been diagnosed as being critically ill and are under medical treatment, wherein said comparing is carried out in a computer processor using computer executable code.

The methods of the present invention may in part be computer-implemented. For example, the step of comparing the detected level of a marker, e.g. the proADM or fragments thereof, with a reference level can be performed in a computer system. In the computer-system, the determined level of the marker(s) can be combined with other marker levels and/or parameters of the subject in order to calculate a score, which is indicative for the diagnosis, prognosis, prediction, risk assessment and/or risk stratification. For example, the determined values may be entered (either manually by a health professional or automatically from the device(s) in which the respective marker level(s) has/have been determined) into the computer-system. The computer-system can be directly at the point-of-care (e.g. primary care, ICU or ED) or it can be at a remote location connected via a computer network (e.g. via the internet, or specialized medical cloud-systems, optionally combinable with other IT-systems or platforms such as hospital information systems (HIS)). Typically, the computer-system will store the values (e.g. marker level or parameters such as age, blood pressure, weight, sex, etc. or clinical scoring systems such as SOFA, qSOFA, BMI etc.) on a computer-readable medium and calculate the score based-on pre-defined and/or prestored reference levels or reference values. The resulting score will be displayed and/or printed for the user (typically a health professional such as a physician). Alternatively or in addition, the associated prognosis, diagnosis, assessment, treatment guidance, patient management guidance or stratification will be displayed and/or printed for the user (typically a health professional such as a physician).

In one embodiment of the invention, a software system can be employed, in which a machine learning algorithm is evident, preferably to identify hospitalized patients at risk for sepsis, severe sepsis and septic shock using data from electronic health records (EHRs). A machine learning approach can be trained on a random forest classifier using EHR data (such as labs, biomarker expression, vitals, and demographics) from patients. Machine learning is a type of artificial intelligence that provides computers with the ability to learn complex patterns in data without being explicitly programmed, unlike simpler rule-based systems. Earlier studies have used electronic health record data to trigger alerts to detect clinical deterioration in general. In one embodiment of the invention the processing of proADM levels may be incorporated into appropriate software for comparison to existing data sets, for example proADM levels may also be processed in machine learning software to assist in diagnosing or prognosing the occurrence of an adverse event.

The combined employment of proADM or fragments thereof in combination with another biomarker such as PCT or CRP may be realized either in a single multiplex assay, or in two separate assays conducted on a sample form the patient. The sample may relate to the same sample, or to different samples. The assay employed for the detection and determination of proADM and for example PCT may also be the same or different, for example an immunoassay may be employed for the determination of one of the above markers. More detailed descriptions of suitable assays are provided below.

Cut-off values and other reference levels of proADM or fragments thereof in patients who have been diagnosed as being critically ill and are under treatment may be determined by previously described methods. For example, methods are known to a skilled person for using the Coefficient of variation in assessing variability of quantitative assays in order to establish reference values and/or cut-offs (George F. Reed et al., Clin Diagn Lab Immunol.2002; 9(6):1235-1239).

Additionally, functional assay sensitivity can be determined in order to indicate statistically significant values for use as reference levels or cut-offs according to established techniques. Laboratories are capable of independently establishing an assay's functional sensitivity by a clinically relevant protocol. "Functional sensitivity" can be considered as the concentration that results in a coefficient of variation (CV) of 20% (or some other predetermined % CV), and is thus a measure of an assay's precision at low analyte levels. The CV is therefore a standardization of the standard deviation (SD) that allows comparison of variability estimates regardless of the magnitude of analyte concentration, at least throughout most of the working range of the assay.

Furthermore, methods based on ROC analysis can be used to determine statistically significant differences between two clinical patient groups. Receiver Operating Characteristic (ROC) curves measure the sorting efficiency of the model's fitted probabilities to sort the response levels. ROC curves can also aid in setting criterion points in diagnostic tests. The higher the curve from the diagonal, the better the fit. If the logistic fit has more than two response levels, it produces a generalized ROC curve. In such a plot, there is a curve for each response level, which is the ROC curve of that level versus all other levels. Software capable of enabling this kind of analysis in order to establish suitable reference levels and cut-offs is available, for example JMP 12, JMP 13, Statistical Discovery, from SAS.

Cut off values may similarly be determined for PCT. Literature is available to a skilled person for determining an appropriate cut-off, for example Philipp Schuetz et al. (BMC Medicine. 2011; 9:107) describe that at a cut-off of 0.1 ng/mL, PCT had a very high sensitivity to exclude infection. Terence Chan et al. (Expert Rev.Mol.Diagn.2011; 11(5), 487.496) described that indicators such as the positive and negative likelihood ratios, which are calculated based on sensitivity and specificity, are also useful for assessing the strength of a diagnostic test. Values are commonly graphed for multiple cut-off values (CVs) as a receiver operating characteristic curve. The area under the curve value is used to determine the best diagnostically relevant CV. This literature describes the variation of CVs (cut-off values, that is dependent on the assay and study design), and suitable methods for determining cut-off values.

Population averages levels of proADM or fragments thereof may also be used as reference values, for example mean proADM population values, whereby patients that are diagnosed as critically ill may be compared to a control population, wherein the control group preferably comprises more than 10, 20, 30, 40, 50 or more subjects.

In one embodiment of the invention, the cut-off level for PCT may be a value in the range of 0.01 to 100.00 ng/mL in a serum sample, when using for example a Luminex MAC Pix E-Bioscience Assay or the BRAHMS PCT-Kryptor Assay. In a preferred embodiment the cut-off level of PCT may be in the range of 0.01 to 100, 0.05 to 50, 0.1 to 20, or 0.1 to 2 ng/mL, and most preferably >0,05 to 0,5 ng/mL. Any value within these ranges may be considered as an appropriate cut-off value. For example, 0.01, 0.05, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 3, 4, 5, 6, 7, 8, 9, 10, 15, 20, 25, 30, 35, 40, 45, 50, 55, 60, 65, 70, 75, 80, 85, 90, 95 or 100 ng/mL may be employed. In some embodiments, PCT levels for healthy subjects are approximately 0.05 ng/mL.

### DETAILED DESCRIPTION OF THE INVENTION

The present invention is based on data disclosed herein that demonstrates that proADM enables effective prognostic statements regarding whether hospitalization is required, thereby providing effective means for therapy guidance in a patient with symptoms of an infectious disease.

The present invention has the following advantages over the conventional methods: the inventive methods and the kits are fast, objective, easy to use and precise. The methods and kits of the invention relate to markers and clinical scores that are easily measurable in routine methods, because the levels of proADM, PCT, lactate, c-reactive protein, SOFA, qSOFA, APACHE II, SAPS II can be determined in routinely obtained blood samples or further biological fluids or samples obtained from a subject.

As used herein, the "patient" or "subject" may be a vertebrate. In the context of the present invention, the term "subject" includes both humans and animals, particularly mammals, and other organisms.

In the sense of the present invention, a patient with symptoms of an infectious disease is a subject who presents with one or more of, without limitation to, fever, diarrhea, fatigue, muscle aches, coughing, if have been bitten by an animal, having trouble breathing, severe headache with fever, rash or swelling, unexplained or prolonged fever or vision problems. Other symptoms may be fever and chills, very low body temperature, peeing less than normal, rapid pulse, rapid breathing, nausea and vomiting. In preferred embodiments the symptoms of an infectious disease are fever, diarrhea, fatigue, muscle aches, rapid pulse, rapid breathing, nausea and vomiting and/or coughing.

As used herein "infectious disease" comprises all diseases or disorders that are associated with bacterial and/or viral and/or fungal infections.

As used herein, "diagnosis" in the context of the present invention relates to the recognition and (early) detection of a clinical condition. Also the assessment of the severity may be encompassed by the term "diagnosis".

"Prognosis" relates to the prediction of an outcome or a specific risk for a subject. This may also include an estimation of the chance of recovery or the chance of an adverse outcome for said subject.

The methods of the invention may also be used for monitoring, therapy monitoring, therapy guidance and/or therapy control. "Monitoring" relates to keeping track of a patient and potentially occurring complications, e.g. to analyze the progression of the healing process or the influence of a particular treatment or therapy on the health state of the patient.

The term "therapy monitoring" or "therapy control" in the context of the present invention refers to the monitoring and/or adjustment of a therapeutic treatment of said patient, for example by obtaining feedback on the efficacy of the therapy. As used herein, the term "therapy guidance" refers to application of certain therapies, therapeutic actions or medical interventions based on the value/level of one or more biomarkers and/or clinical parameter and/or clinical scores. This includes the adjustment of a therapy or the discontinuation of a therapy.

In the present invention, the terms "risk assessment" and "risk stratification" relate to the grouping of subjects into different risk groups according to their further prognosis. Risk assessment also relates to stratification for applying preventive and/or therapeutic measures. The term "therapy stratification" in particular relates to grouping or classifying patients into different groups, such as risk groups or therapy groups that receive certain differential therapeutic measures depending on their classification. The term "therapy stratification" also relates to grouping or classifying patients with infections or having symptoms of an infectious disease into a group that are not in need to receive certain therapeutic measures.

It is understood that in the context of the present invention "determining the level of proADM or fragment(s) thereof" or the like refers to any means of determining proADM or a fragment thereof. The fragment can have any length, e.g. at least about 5, 10, 20, 30, 40, 50 or 100 amino acids, so long as the fragment allows the unambiguous determination of the level of proADM or fragment thereof. In particular preferred aspects of the invention, "determining the level of proADM" refers to determining the level of midregional proadrenomedullin (MR-proADM). MR-proADM is a fragment and/or region of proADM.

The peptide adrenomedullin (ADM) was discovered as a hypotensive peptide comprising 52 amino acids, which had been isolated from a human phenochromocytome (Kitamura et al., 1993). Adrenomedullin (ADM) is encoded as a precursor peptide comprising 185 amino acids ("preproadrenomedullin" or "pre proADM"). An exemplary amino acid sequence of ADM is given in SEQ ID NO: 1.
SEQ ID NO:1: amino acid sequence of pre-pro-ADM:

1 MKLVSVALMY LGSLAFLGAD TARLDVASEF RKKWNKWALS RGKRELRMSS
51 SYPTGLADVK AGPAQTLIRP QDMKGASRSP EDSSPDAARI RVKRYRQSMN
101 NFQGLRSFGC RFGTCTVQKL AHQIYQFTDK DKDNVAPRSK ISPQGYGRRR
151 RRSLPEAGPG RTLVSSKPQA HGAPAPPSGS APHFL

ADM comprises the positions 95-146 of the pre-proADM amino acid sequence and is a splice product thereof. "Proadrenomedullin" ("proADM") refers to pre-proADM without the signal sequence (amino acids 1 to 21), i.e. to amino acid residues 22 to 185 of pre-proADM. "Midregional proadrenomedullin" ("MR-proADM") refers to the amino acids 42 to 95 of pre-proADM. An exemplary amino acid sequence of MR-proADM is given in SEQ ID NO: 2.

SEQ ID NO:2: amino acid sequence of MR-pro-ADM (AS 45-92 of pre-pro-ADM):
ELRMSSSYPT GLADVKAGPA QTLIRPQDMK GASRSPEDSS PDAARIRV

It is also envisaged herein that a peptide and fragment thereof of pre-proADM or MR-proADM can be used for the herein described methods. For example, the peptide or the fragment thereof can comprise the amino acids 22-41 of pre-proADM (PAMP peptide) or amino acids 95-146 of pre-proADM (mature adrenomedullin, including the biologically active form, also known as bio-ADM). A C-terminal fragment of proADM (amino acids 153 to 185 of pre proADM) is called adrenotensin. Fragments of the proADM peptides or fragments of the MR-proADM can comprise, for example, at least about 5, 10, 20, 30 or more amino acids. Accordingly, the fragment of proADM may, for example, be selected from the group consisting of MR-proADM, PAMP, adrenotensin and mature adrenomedullin, preferably herein the fragment is MR-proADM.

The determination of these various forms of ADM or proADM and fragments thereof also encompass measuring and/or detecting specific sub-regions of these molecules, for example by employing antibodies or other affinity reagents directed against a particular portion of the molecules, or by determining the presence and/or quantity of the molecules by measuring a portion of the protein using mass spectrometry. Any one or more of the "ADM peptides or fragments" described herein may be employed in the present invention.

Accordingly, the methods and kits of the present invention can also comprise determining at least one further biomarker, marker, clinical score and/or parameter in addition to ADM.

As used herein, a parameter is a characteristic, feature, or measurable factor that can help in defining a particular system. A parameter is an important element for health- and physiology-related assessments, such as a disease/disorder/clinical condition risk, preferably organ dysfunction(s). Furthermore, a parameter is defined as a characteristic that is objectively measured and evaluated as an indicator of normal biological processes, pathogenic processes, or pharmacologic responses to a therapeutic intervention. An exemplary parameter can be selected from the group consisting of Acute Physiology and Chronic Health Evaluation II (APACHE II), the simplified acute physiology score (SAPSII score), sequential organ failure assessment score (SOFA score), quick sequential organ failure assessment score (qSOFA), body mass index, weight, age, sex, IGS II, liquid intake, white blood cell count, sodium, potassium, temperature, blood pressure, dopamine, bilirubin, respiratory rate, partial pressure of oxygen, World Federation of Neurosurgical Societies (WFNS) grading, and Glasgow Coma Scale (GCS).

As used herein, terms such as "marker", "surrogate", "prognostic marker", "factor" or "biomarker" or "biological marker" are used interchangeably and relate to measurable and quantifiable biological markers (e.g., specific protein or enzyme concentration or a fragment thereof, specific hormone concentration or a fragment thereof, or presence of biological substances or a fragment thereof) which serve as indices for health- and physiology-related assessments, such as a disease/disorder/clinical condition risk, preferably an adverse event. A marker or biomarker is defined as a characteristic that can be objectively measured and evaluated as an indicator of normal biological processes, pathogenic processes, or pharmacologic responses to a therapeutic intervention. Biomarkers may be measured in a sample (as a blood, plasma, urine, or tissue test).

The at least one further marker and/or parameter of said subject can be selected from the group consisting of a level of lactate in said sample, a level of procalcitonin (PCT) in said sample, the sequential organ failure assessment score (SOFA score) of said subject, optionally the quick SOFA score, the simplified acute physiology score (SAPSII) of said subject, the Acute Physiology and Chronic Health Evaluation II (APACHE II) score of said subject and a level of the soluble fms-like tyrosine kinase-1 (sFlt-1), Histone H2A, Histone H2B, Histone H3, Histone H4, calcitonin, Endothelin-1 (ET-1), Arginine Vasopressin (AVP), Atrial Natriuretic Peptide (ANP), Neutrophil Gelatinase-Associated Lipocalin (NGAL), Troponin, Brain Natriuretic Peptide (BNP), C-Reactive Protein (CRP), Pancreatic Stone Protein (PSP), Triggering Receptor Expressed on Myeloid Cells 1 (TREM1), Interleukin-6 (IL-6), Interleukin-1, Interleukin-24 (IL-24), Interleukin-22 (IL-22), Interleukin (IL-20) other ILs, Presepsin (sCD14-ST), Lipopolysaccharide Binding Protein (LBP), Alpha-1-Antitrypsin, Matrix Metalloproteinase 2 (MMP2), Metalloproteinase 2 (MMP8), Matrix Metalloproteinase 9 (MMP9), Matrix Metalloproteinase 7 (MMP7, Placental growth factor (PIGF), Chromogranin A, S100A protein, S100B protein and Tumor Necrosis Factor α (TNFα), Neopterin, Alpha-1-Antitrypsin, pro-arginine vasopressin (AVP, proAVP or Copeptin), procalcitonin, atrial natriuretic peptide (ANP, pro-ANP), Endothelin-1, CCL1/TCA3, CCL11, CCL12/MCP-5, CCL13/MCP-4, CCL14, CCL15, CCL16, CCL17/TARC, CCL18, CCL19, CCL2/MCP-1, CCL20, CCL21, CCL22/MDC, CCL23, CCL24, CCL25, CCL26, CCL27, CCL28, CCL3, CCL3L3, CCL4, CCL4L1/LAG-1, CCL5, CCL6, CCL7, CCL8, CCL9, CX3CL1, CXCL1, CXCL10, CXCL11, CXCL12, CXCL13, CXCL14, CXCL15, CXCL16, CXCL17, CXCL2/MIP-2, CXCL3, CXCL4, CXCL5, CXCL6, CXCL7/Ppbp, CXCL9, IL8/CXCL8, XCL1, XCL2, FAM19A1, FAM19A2, FAM19A3, FAM19A4, FAM19A5, CLCF1, CNTF, IL11, IL31, IL6, Leptin, LIF, OSM, IFNA1, IFNA10, IFNA13, IFNA14, IFNA2, IFNA4, IFNA7, IFNB1, IFNE, IFNG, IFNZ, IFNA8, IFNA5/IFNaG, IFNw/IFNW1, BAFF, 4-1BBL, TNFSF8, CD40LG, CD70, CD95L/CD178, EDA-A1, TNFSF14, LTA/TNFB, LTB, TNFa, TNFSF10, TNFSF11, TNFSF12, TNFSF13, TNFSF15, TNFSF4, IL18, IL18BP, IL1A, IL1B, IL1F10, IL1F3/IL1RA, IL1F5, IL1F6, IL1F7, IL1F8, IL1RL2, IL1F9, IL33 or a fragment thereof. Further markers comprise membrane microparticle, platelet count, mean platelet volume (MPV), sCD14-ST, prothrombinase, antithrombin and/ antithrombin activity, cationic protein 18 (CAP18), von Willebrand factor (vWF)-cleaving proteases, lipoproteins in combination with CRP, fibrinogen, fibrin, B2GP1, GPIIb-IIIa, non-denatured D-dimer of fibrin, platelet factor 4, histones and a PT-Assay.

As used herein, "procalcitonin" or "PCT" relates to a peptide spanning amino acid residues 1-116, 2-116, 3-116, or fragments thereof, of the procalcitonin peptide. PCT is a peptide precursor of the hormone calcitonin. Thus the length of procalcitonin fragments is at least 12 amino acids, preferably more than 50 amino acids, more preferably more than 110 amino acids. PCT may comprise post-translational modifications such as glycosylation, liposidation or derivatization. Procalcitonin is a precursor of calcitonin and katacalcin. Thus, under normal conditions the PCT levels in the circulation are very low (< about 0.05 ng/ml).

The level of PCT in the sample of the subject can be determined by immunoassays as described herein. As used herein, the level of ribonucleic acid or deoxyribonucleic acids encoding "procalcitonin" or "PCT" can also be determined. Methods for the determination of PCT are known to a skilled person, for example by using products obtained from Thermo Fisher Scientific / B·R·A·H·M·S GmbH.

Lactate, or lactic acid, is an organic compound with the formula CH₃CH(OH)COOH, which occurs in bodily fluids including blood. Blood tests for lactate are performed to determine the status of the acid base homeostasis in the body. Lactic acid is a product of cell metabolism that can accumulate when cells lack sufficient oxygen (hypoxia) and must turn to a less efficient means of energy production, or when a condition causes excess production or impaired clearance of lactate. Lactic acidosis can be caused by an inadequate amount of oxygen in cells and tissues (hypoxia), for example if someone has a condition that may lead to a decreased amount of oxygen delivered to cells and tissues, such as shock, septic shock or congestive heart failure, the lactate test can be used to help detect and evaluate the severity of hypoxia and lactic acidosis.
C-reactive protein (CRP) is a pentameric protein, which can be found in bodily fluids such as blood plasma. CRP levels can rise in response to inflammation. Measuring and charting CRP values can prove useful in determining disease progress or the effectiveness of treatments.

As used herein, the "sequential organ failure assessment score" or "SOFA score" is one score used to track a patient's status during the stay in an intensive care unit (ICU). The SOFA score is a scoring system to determine the extent of a person's organ function or rate of failure. The score is based on six different scores, one each for the respiratory, cardiovascular, hepatic, coagulation, renal and neurological systems. Both the mean and highest SOFA scores being predictors of outcome. An increase in SOFA score during the first 24 to 48 hours in the ICU predicts a mortality rate of at least 50% up to 95%. Scores less than 9 give predictive mortality at 33% while above 14 can be close to or above 95%.

As used herein, the quick SOFA score (qSOFA) is a scoring system that indicates a patient's organ dysfunction or mortality risk. The score is based on three criteria: 1) an alteration in mental status, 2) a decrease in systolic blood pressure of less than 100 mm Hg, 3) a respiration rate greater than 22 breaths per minute. Patients with two or more of these conditions are at greater risk of having an organ dysfunction or to die. A "positive" qSOFA Score (≥2) suggests high risk of poor outcome in patients with suspected infection. These patients should be more thoroughly assessed for evidence of organ dysfunction.

As used herein, "APACHE II" or "Acute Physiology and Chronic Health Evaluation II" is a severity-of-disease classification scoring system (Knaus et al., 1985). It can be applied within 24 hours of admission of a patient to an intensive care unit (ICU) and may be determined based on 12 different physiologic parameters: AaDO2 or PaO2 (depending on FiO2), temperature (rectal), mean arterial pressure, pH arterial, heart rate, respiratory rate, sodium (serum), potassium (serum), creatinine, hematocrit, white blood cell count and Glasgow Coma Scale.

As used herein, "SAPS II" or "Simplified Acute Physiology Score II" relates to a system for classifying the severity of a disease or disorder (see Le Gall JR et al., A new Simplified Acute Physiology Score (SAPS II) based on a European/North American multicenter study. JAMA. 1993;270(24):2957-63.). The SAPS II score is made of 12 physiological variables and 3 disease-related variables. The point score is calculated from 12 routine physiological measurements, information about previous health status and some information obtained at admission to the ICU. The SAPS II score can be determined at any time, preferably, at day 2. The "worst" measurement is defined as the measure that correlates to the highest number of points. The SAPS II score ranges from 0 to 163 points. The classification system includes the followings parameters: Age, Heart Rate, Systolic Blood Pressure, Temperature, Glasgow Coma Scale, Mechanical Ventilation or CPAP, PaO2, FiO2, Urine Output, Blood Urea Nitrogen, Sodium, Potassium, Bicarbonate, Bilirubin, White Blood Cell, Chronic diseases and Type of admission. There is a sigmoidal relationship between mortality and the total SAPS II score. The mortality of a subject is 10% at a SAPSII score of 29 points, the mortality is 25% at a SAPSII score of 40 points, the mortality is 50% at a SAPSII score of 52 points, the mortality is 75% at a SAPSII score of 64 points, the mortality is 90% at a SAPSII score of 77 points (Le Gall loc. cit.).

As used herein, the term "sample" is a biological sample that is obtained or isolated from the patient or subject. "Sample" as used herein may, e.g., refer to a sample of bodily fluid or tissue obtained for the purpose of analysis, diagnosis, prognosis, or evaluation of a subject of interest, such as a patient. Preferably herein, the sample is a sample of a bodily fluid, such as blood, serum, plasma, cerebrospinal fluid, urine, saliva, sputum, pleural effusions, cells, a cellular extract, a tissue sample, a tissue biopsy, a stool sample and the like. Particularly, the sample is blood, blood plasma, blood serum.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood), for example for at least 15 minutes at 2000 to 3000 g.

"Serum" in the context of the present invention is the liquid fraction of whole blood that is collected after the blood is allowed to clot. When coagulated blood (clotted blood) is centrifuged serum can be obtained as supernatant.

As used herein, "urine" is a liquid product of the body secreted by the kidneys through a process called urination (or micturition) and excreted through the urethra.

In embodiments of the present invention the condition requiring hospitalization may be sepsis, severe sepsis and/or septic shock. "Sepsis" in the context of the invention refers to a systemic response to infection. Alternatively, sepsis may be seen as the combination of SIRS with a confirmed infectious process or an infection. Sepsis may be characterized as clinical syndrome defined by the presence of both infection and a systemic inflammatory response (Levy MM et al. 2001 SCCM/ESICM/ACCP/ATS/SIS International Sepsis Definitions Conference. Crit Care Med. 2003 Apr;31(4):1250-6). The term "sepsis" used herein includes, but is not limited to, sepsis, severe sepsis, and septic shock.

The term "sepsis" used herein includes, but is not limited to, sepsis, severe sepsis, and septic shock. Severe sepsis in refers to sepsis associated with organ dysfunction, hypoperfusion abnormality, or sepsis-induced hypotension. Hypoperfusion abnormalities include lactic acidosis, oliguria and acute alteration of mental status. Sepsis-induced hypotension is defined by the presence of a systolic blood pressure of less than about 90 mm Hg or its reduction by about 40 mm Hg or more from baseline in the absence of other causes for hypotension (e.g. cardiogenic shock). Septic shock is defined as severe sepsis with sepsis-induced hypotension persisting despite adequate fluid resuscitation, along with the presence of hypoperfusion abnormalities or organ dysfunction (Bone et al., CHEST 101(6): 1644-55, 1992).

The term sepsis may alternatively be defined as life-threatening organ dysfunction caused by a dysregulated host response to infection. For clinical operationalization, organ dysfunction can preferably be represented by an increase in the Sequential Organ Failure Assessment (SOFA) score of 2 points or more, which is associated with an in-hospital mortality greater than 10%. Septic shock may be defined as a subset of sepsis in which particularly profound circulatory, cellular, and metabolic abnormalities are associated with a greater risk of mortality than with sepsis alone. Patients with septic shock can be clinically identified by a vasopressor requirement to maintain a mean arterial pressure of 65 mm Hg or greater and serum lactate level greater than 2 mmol/L (>18 mg/dL) in the absence of hypovolemia.

The term "sepsis" used herein relates to all possible stages in the development of sepsis. The term "sepsis" also includes severe sepsis or septic shock based on the SEPSIS-2 definition (Bone et al., 2009). The term "sepsis" also includes subjects falling within the SEPSIS-3 definition (Singer et al., 2016). The term "sepsis" used herein relates to all possible stages in the development of sepsis.

As used herein, "infection" within the scope of the invention means a pathological process caused by the invasion of normally sterile tissue or fluid by pathogenic or potentially pathogenic agents/ pathogens, organisms and/or microorganisms, and relates preferably to infection(s) by bacteria, viruses, fungi, and/or parasites. Accordingly, the infection can be a bacterial infection, viral infection, and/or fungal infection. The infection can be a local or systemic infection. For the purposes of the invention, a viral infection may be considered as infection by a microorganism.

Furthermore, the infection-related complication can be a "nosocomial" infection. Nosocomial infections are also called hospital-acquired infections (HAI) are infections that are acquired in a hospital or other health care facility. To emphasize both hospital and nonhospital settings, it is sometimes instead called a health care-associated infection (HAI or HCAI). Such an infection can be acquired in hospital, nursing home, rehabilitation facility, outpatient clinic, or other clinical settings. Nosocomial infection may be spread to the susceptible patient in the clinical setting by various means. Health care staff can spread infection, in addition to contaminated equipment, bed linens, or air droplets. The infection can originate from the outside environment, another infected patient, staff that may be infected, or in some cases, the source of the infection cannot be determined. In some cases the microorganism originates from the patient's own skin microbiota, becoming opportunistic after surgery or other procedures that compromise the protective skin barrier. Though the patient may have contracted the infection from their own skin, the infection is still considered nosocomial since it develops in the health care setting.

Further, the subject suffering from an infection can suffer from more than one source(s) of infection simultaneously. For example, the subject suffering from an infection can suffer from a bacterial infection and viral infection; from a viral infection and fungal infection; from a bacterial and fungal infection, and from a bacterial infection, fungal infection and viral infection, or suffer from a mixed infection comprising one or more of the infections listed herein, including potentially a superinfection, for example one or more bacterial infections in addition to one or more viral infections and/or one or more fungal infections.

As used herein "infectious disease" comprises all diseases or disorders that are associated with bacterial and/or viral and/or fungal infections. Preferably the infectious disease is bacterial.

In the context of the present invention, the term "medical treatment" or "treatment" comprises various treatments and therapeutic strategies, which comprise, without limitation, anti-inflammatory strategies, administration of ADM-antagonists such as therapeutic antibodies, si-RNA or DNA, the extracorporal blood purification or the removal of harmful substances via apheresis, dialyses, adsorbers to prevent the cytokine storm, removal of inflammatory mediators, plasma apheresis, administration of vitamines such as vitamin C, surgery, emergency surgery, ventilation like mechanical ventilation and non-mechanical ventilation, to provide the body with sufficient oxygen, for example, focus cleaning procedures, transfusion of blood products, infusion of colloids, organ replacement, such as renal or liver replacement, antibiotic treatment, invasive mechanical ventilation, non-invasive mechanical ventilation, vasopressor use, fluid therapy, apheresis and measures for organ protection.

A skilled person is capable of determining which of the treatments described herein require administration in a hospital setting.

A skilled person is also capable of determining which medical conditions, and which degrees of severity of such medical conditions, require treatments only (or primarily) available in hospital settings, for example in the ED or ICU.

Further treatments of the present invention comprise the administration of cells or cell products like stem cells, blood or plasma, and the stabilization of the patients circulation and the protection of endothelial glycocalyx, for example via optimal fluid management strategies, for example to reach normovolemia and prevent or treat hypervolemia or hypovolemia. Moreover, vasopressors or e.g. catecholamine as well as albumin or heparanase inhibition via unfractionated heparin or N-desulfated re-N-acetylated heparin are useful treatments to support the circulation and endothelial layer.

Additionally, medical treatments of the present invention comprise, without limitation, stabilization of the blood clotting, anti-fibrinolytic treatment, iNOS inhibitors, anti-inflammatory agents like hydrocortisone, sedatives and analgetics as well as insuline.

Artificial and mechanical ventilation are effective approaches to enhance proper gas exchange and ventilation and aim to save life during severe hypoxemia. Artificial ventilation relates to assisting or stimulating respiration of the subject. Artificial ventilation may be selected from the group consisting of mechanical ventilation, manual ventilation, extracorporeal membrane oxygenation (ECMO) and noninvasive ventilation (NIV). Mechanical ventilation relates to a method to mechanically assist or replace spontaneous breathing. This may involve a machine called a ventilator. Mechanical ventilation may be High-Frequency Oscillatory Ventilation or Partial Liquid Ventilation.

"Renal replacement therapy" (RRT) relates to a therapy that is employed to replace the normal blood-filtering function of the kidneys. Renal replacement therapy may refer to dialysis (e.g. hemodialysis or peritoneal dialysis), hemofiltration, and hemodiafiltration. Such techniques are various ways of diverting the blood into a machine, cleaning it, and then returning it to the body. Renal replacement therapy may also refer to kidney transplantation, which is the ultimate form of replacement in that the old kidney is replaced by a donor kidney. The hemodialysis, hemofiltration, and hemodiafiltration may be continuous or intermittent and can use an arteriovenous route (in which blood leaves from an artery and returns via a vein) or a venovenous route (in which blood leaves from a vein and returns via a vein). This results in various types of RRT. For example, the renal replacement therapy may be selected from the group of, but not limited to continuous renal replacement therapy (CRRT), continuous hemodialysis (CHD), continuous arteriovenous hemodialysis (CAVHD), continuous venovenous hemodialysis (CVVHD), continuous hemofiltration (CHF), continuous arteriovenous hemofiltration (CAVH or CAVHF), continuous venovenous hemofiltration (CVVH or CVVHF), continuous hemodiafiltration (CHDF), continuous arteriovenous hemodiafiltration (CAVHDF), continuous venovenous hemodiafiltration (CVVHDF), intermittent renal replacement therapy (IRRT), intermittent hemodialysis (IHD), intermittent venovenous hemodialysis (IVVHD), intermittent hemofiltration (IHF), intermittent venovenous hemofiltration (IVVH or IVVHF), intermittent hemodiafiltration (IHDF) and intermittent venovenous hemodiafiltration (IVVHDF).

Medical treatment also comprises methods to avoid hypothermia which includes the use of warmed intravenous fluids and warm air blankets.

Medical treatment also comprises wound management including haemorrhage control, bleeding control techniques with or without tourniquets, wound cleaning, local anaesthetic application, wound closure techniques where skin adhesive strips, tissue adhesive glue, sutures, staples and wound dressings can be used.

The term "fluid therapy" as used in the present invention relates to the administration of a liquid to a subject in need thereof. The terms "fluid replacement" or "fluid resuscitation" may also be used. Fluid therapy comprises but is not limited to fluids being replaced with oral rehydration therapy (drinking), intravenous therapy, rectally, or the injection of fluid into subcutaneous tissue. Intravenous methods are preferred. "Colloids" are dispersions of large organic molecules (e.g., Gelofusin, Voluven). Colloids are typically suspensions of molecules within a carrier solution that are relatively incapable of crossing the healthy semipermeable capillary membrane due to their molecular weight. In one embodiment, the method indicates that the fluid therapy comprises a colloid selected from gelatin, albumin and/or starch solution, or blood or a fluid derived from blood. "Crystalloids" are solutions of small molecules in water (e.g., sodium chloride, glucose, Hartmann's solution, or Ringer's solution). Crystalloids are typically solutions of ions that are freely permeable but contain concentrations of sodium and chloride that determine the tonicity of the fluid.

"Fluid management" refers to the monitoring and controlling of the fluid status of a subject and the administration of fluids to stabilize the circulation or organ vitality, by e.g. oral, enteral or intravenous fluid administration. It comprises the stabilization of the fluid and electrolyte balance or the prevention or correction of hyper- or hypovolemia as well as the supply of blood products.

Cleaning Procedures are hygienic methods to prevent subjects from infections, especially nosocomial infections, comprising disinfection of all organic and inorganic surfaces that could get in contact with a patient, such as for example, skin, objects in the patient's room, medical devices, diagnostic devices, or room air. Cleaning procedures include the use of protective clothes and units, such as mouth guards, gowns, gloves or hygiene lock, and actions like restricted patient visits. Furthermore, cleaning procedures comprise the cleaning of the patient itself and the clothes or the patient.

In a preferred embodiment, the term "medical treatment" or "treatment" comprises antibiotic treatment such as intravenous antibiotic, oral antibiotics or topical antibiotics.

A medical treatment of the present invention may be an antibiotic treatment, wherein one or more "antibiotics" or "antibiotic agents" may be administered if an infection has been diagnosed or prognosed by the method of the invention. Antibiotics or antibiotic agents according to the present invention also encompass potentially the anti-fungal or anti-viral compounds used to treat a diagnosed infection or sepsis. The antibiotic agents commonly applied in the treatment of any given infection, as separated into the classes of pathogen are:

Gram positive coverage: Penicillins, (ampicillin, amoxicillin), penicillinase resistant, (Dicloxacillin, Oxacillin), Cephalosporins (1st and 2nd generation), Macrolides (Erythromycin, Clarithromycin, Azithromycin), Quinolones (gatifloxacin, moxifloxacin, levofloxacin), Vancomycin, Sulfonamide/trimethoprim, Clindamycin, Tetracyclines, Chloramphenicol, Linezolid, Synercid.

Gram negative coverage: Broad spectrum penicillins (Ticarcillin, clavulanate, piperacillin, tazobactam), Cephalosporins (2nd, 3rd, and 4th generation), Aminoglycosides, Macrolides, Azithromycin, Quinolones (Ciprofloxacin), Monobactams (Azetreonam), Sulfonamide/trimethoprim, Carbapenems (Imipenem), Chloramphenicol.

Pseudomonas coverage: Ciprofloxacin, Aminoglycosides, Some 3rd generation cephalosporins, 4th generation cephalosporins, Broad spectrum penicillins, Carbapenems.

Fungal treatments: Allyamines, Amphotericin B, Fluconazole and other Azoles, itraconazole, voriconazole, posaconazole, ravuconazole, echinocandins, Flucytosine, sordarins, chitin synthetase inhibitors, topoisomerase inhibitors, lipopeptides, pradimycins, Liposomal nystatin, Voriconazole, Echinocanidins, Imidazole, Triazole, Thiazole, Polyene.

Anti-viral treatments: Abacavir, Acyclovir (Aciclovir), activated caspase oligomerizer, Adefovir, Amantadine , Amprenavir(Agenerase), Ampligen, Arbidol, Atazanavir, Atripla , Balavir, Cidofovir, Combivir, Dolutegravir, Darunavir, Delavirdine, Didanosine, Double-stranded RNA, Docosanol, Edoxudine, Efavirenz, Emtricitabine, Enfuvirtide, Entecavir, Ecoliever, Famciclovir, Fixed dose combination (antiretroviral), Fomivirsen, Fosamprenavir, Foscarnet, Fosfonet, Fusion inhibitor, Ganciclovir, Ibacitabine, Imunovir, Idoxuridine, Imiquimod, Indinavir, Inosine, Integrase inhibitor, Interferon type III, Interferon type II, Interferon type I, Interferon, Lamivudine, Lopinavir, Loviride, Maraviroc, Moroxydine, Methisazone, Morpholinos, Nelfinavir, Nevirapine, Nexavir, Nitazoxanide, Nucleoside analogues, Novir, Oseltamivir (Tamiflu), Peginterferon alfa-2a, Penciclovir, Peramivir, Pleconaril, Podophyllotoxin, Protease inhibitor (pharmacology), Raltegravir, Reverse transcriptase inhibitor, Ribavirin, Ribozymes, Rifampicin, Rimantadine, Ritonavir, RNase H, protease inhibitors, Pyramidine, Saquinavir, Sofosbuvir, Stavudine, Synergistic enhancer (antiretroviral), Telaprevir, Tenofovir, Tenofovir disoproxil, Tipranavir, Trifluridine, Trizivir, Tromantadine, Truvada, Valaciclovir (Valtrex), Valganciclovir, Vicriviroc, Vidarabine, Viramidine, Zalcitabine, Zanamivir (Relenza), Zidovudine.

Furthermore, antibiotic agents comprise bacteriophages for treatment of bacterial infections, synthetic antimicrobial peptides or iron-antagonists/iron chelator. Also, therapeutic antibodies or antagonist against pathogenic structures like anti-VAP-antibodies, anti-resistant clone vaccination, administration of immune cells, such as in vitro primed or modulated T-effector cells, are antibiotic agents that represent treatment options for critically ill patients, such as sepsis patients. Further antibiotic agents/treatments or therapeutic strategies against infection or for the prevention of new infections include the use of antiseptics, decontamination products, anti-virulence agents like liposomes, sanitation, wound care, surgery.

It is also possible to combine several of the aforementioned antibiotic agents or treatments strategies.

According to the present invention proADM and optionally PCT and/or other markers or clinical scores are employed as markers for the diagnosis, prognosis, prediction, risk assessment and/or risk stratification of a patient in developing a medical condition that requires treatment in hospital who presents with symptoms of an infectious disease.

A skilled person is capable of obtaining or developing means for the identification, measurement, determination and/or quantification of any one of the above ADM molecules, or fragments or variants thereof, as well as the other markers of the present invention according to standard molecular biological practice.

The level of proADM or fragments thereof as well as the levels of other markers of the present invention can be determined by any assay that reliably determines the concentration of the marker. Particularly, mass spectrometry (MS) and/or immunoassays can be employed as exemplified in the appended examples. As used herein, an immunoassay is a biochemical test that measures the presence or concentration of a macromolecule/polypeptide in a solution through the use of an antibody or antibody binding fragment or immunoglobulin.

Methods of determining ADM or other the markers such as PCT used in the context of the present invention are intended in the present invention. By way of example, a method may be employed selected from the group consisting of mass spectrometry (MS), luminescence immunoassay (LIA), radioimmunoassay (RIA), chemiluminescence- and fluorescence-immunoassays, enzyme immunoassay (EIA), Enzyme-linked immunoassays (ELISA), luminescence-based bead arrays, magnetic beads based arrays, protein microarray assays, rapid test formats such as for instance immunochromatographic strip tests, rare cryptate assay, and automated systems/analyzers.

Determination of ADM and optionally other markers based on antibody recognition is a preferred embodiment of the invention. As used herein, the term, "antibody" refers to immunoglobulin molecules and immunologically active portions of immunoglobulin (Ig) molecules, i.e., molecules that contain an antigen binding site that specifically binds (immuno reacts with) an antigen. According to the invention, the antibodies may be monoclonal as well as polyclonal antibodies. Particularly, antibodies that are specifically binding to at least proADM or fragments thereof are used.

An antibody is considered to be specific, if its affinity towards the molecule of interest, e.g. ADM, or the fragment thereof is at least 50-fold higher, preferably 100-fold higher, most preferably at least 1000-fold higher than towards other molecules comprised in a sample containing the molecule of interest. It is well known in the art how to develop and to select antibodies with a given specificity. In the context of the invention, monoclonal antibodies are preferred. The antibody or the antibody binding fragment binds specifically to the herein defined markers or fragments thereof. In particular, the antibody or the antibody binding fragment binds to the herein defined peptides of ADM. Thus, the herein defined peptides can also be epitopes to which the antibodies specifically bind. Further, an antibody or an antibody binding fragment is used in the methods and kits of the invention that binds specifically to ADM or proADM, particularly to MR-proADM.

Further, an antibody or an antibody binding fragment is used in the methods and kits of the invention that binds specifically to proADM or fragments thereof and optionally to other markers of the present inventions such as PCT. Exemplary immunoassays can be luminescence immunoassay (LIA), radioimmunoassay (RIA), chemiluminescence- and fluorescence-immunoassays, enzyme immunoassay (EIA), Enzyme-linked immunoassays (ELISA), luminescence-based bead arrays, magnetic beads based arrays, protein microarray assays, rapid test formats, rare cryptate assay. Further, assays suitable for point-of-care testing and rapid test formats such as for instance immune-chromatographic strip tests can be employed. Automated immunoassays are also intended, such as the KRYPTOR assay.

Alternatively, instead of antibodies, other capture molecules or molecular scaffolds that specifically and/or selectively recognize ADM may be encompassed by the scope of the present invention. Herein, the term "capture molecules" or "molecular scaffolds" comprises molecules which may be used to bind target molecules or molecules of interest, i.e. analytes (e.g. ADM, proADM, MR-proADM, and PCT), from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may, for instance, be mediated by ionic, van-der-Waals, pi-pi, sigma-pi, hydrophobic or hydrogen bond interactions or a combination of two or more of the aforementioned interactions or covalent interactions between the capture molecules or molecular scaffold and the target molecules or molecules of interest. In the context of the present invention, capture molecules or molecular scaffolds may for instance be selected from the group consisting of a nucleic acid molecule, a carbohydrate molecule, a PNA molecule, a protein, a peptide and a glycoprotein. Capture molecules or molecular scaffolds include, for example, aptamers, DARpins (Designed Ankyrin Repeat Proteins). Affimers and the like are included.

In certain aspects of the invention, the method is an immunoassay comprising the steps of:
a) contacting the sample with
   i. a first antibody or an antigen-binding fragment or derivative thereof specific for a first epitope of said proADM, and
   ii. a second antibody or an antigen-binding fragment or derivative thereof specific for a second epitope of said proADM; and
b) detecting the binding of the two antibodies or antigen-binding fragments or derivates thereof to said proADM.

Preferably, one of the antibodies can be labeled and the other antibody can be bound to a solid phase or can be bound selectively to a solid phase. In a particularly preferred aspect of the assay, one of the antibodies is labeled while the other is either bound to a solid phase or can be bound selectively to a solid phase. The first antibody and the second antibody can be present dispersed in a liquid reaction mixture, and wherein a first labeling component which is part of a labeling system based on fluorescence or chemiluminescence extinction or amplification is bound to the first antibody, and a second labeling component of said labeling system is bound to the second antibody so that, after binding of both antibodies to said proADM or fragments thereof to be detected, a measurable signal which permits detection of the resulting sandwich complexes in the measuring solution is generated. The labeling system can comprise a rare earth cryptate or chelate in combination with a fluorescent or chemiluminescent dye, in particular of the cyanine type.

In a preferred embodiment, the method is executed as heterogeneous sandwich immunoassay, wherein one of the antibodies is immobilized on an arbitrarily chosen solid phase, for example, the walls of coated test tubes (e.g. polystyrol test tubes; coated tubes; CT) or microtiter plates, for example composed of polystyrol, or to particles, such as for instance magnetic particles, whereby the other antibody has a group resembling a detectable label or enabling for selective attachment to a label, and which serves the detection of the formed sandwich structures. A temporarily delayed or subsequent immobilization using suitable solid phases is also possible.

The method according to the present invention can furthermore be embodied as a homogeneous method, wherein the sandwich complexes formed by the antibody/antibodies and the marker, ADM or a fragment thereof, which is to be detected remains suspended in the liquid phase. In this case it is preferred, that when two antibodies are used, both antibodies are labeled with parts of a detection system, which leads to generation of a signal or triggering of a signal if both antibodies are integrated into a single sandwich. Such techniques are to be embodied in particular as fluorescence enhancing or fluorescence quenching detection methods. A particularly preferred aspect relates to the use of detection reagents which are to be used pair-wise, such as for example the ones which are described in US4882733, EP0180492 or EP0539477 and the prior art cited therein. In this way, measurements in which only reaction products comprising both labeling components in a single immune-complex directly in the reaction mixture are detected, become possible. For example, such technologies are offered under the brand names TRACE® (Time Resolved Amplified Cryptate Emission) or KRYPTOR®, implementing the teachings of the above-cited applications. Therefore, in particular preferred aspects, a diagnostic device is used to carry out the herein provided method. For example, the level of proADM or fragments thereof and/or the level of any further marker of the herein provided method, such as PCT, is determined. In particular preferred aspects, the diagnostic device is KRYPTOR®.

The level of the marker of the present invention, e.g. the proADM or fragments thereof, PCT or fragments thereof, or other markers, can also be determined by a mass spectrometric (MS) based methods. Such a method may comprise detecting the presence, amount or concentration of one or more modified or unmodified fragment peptides of e.g. ADM or the PCT in said biological sample or a protein digest (e.g. tryptic digest) from said sample, and optionally separating the sample with chromatographic methods, and subjecting the prepared and optionally separated sample to MS analysis. For example, selected reaction monitoring (SRM), multiple reaction monitoring (MRM) or parallel reaction monitoring (PRM) mass spectrometry may be used in the MS analysis, particularly to determine the amounts of proADM or fragments thereof.

Herein, the term "mass spectrometry" or "MS" refers to an analytical technique to identify compounds by their mass. In order to enhance the mass resolving and mass determining capabilities of mass spectrometry, the samples can be processed prior to MS analysis. Accordingly, the invention relates to MS detection methods that can be combined with immuno-enrichment technologies, methods related to sample preparation and/or chromatographic methods, preferably with liquid chromatography (LC), more preferably with high performance liquid chromatography (HPLC) or ultra high performance liquid chromatography (UHPLC). Sample preparation methods comprise techniques for lysis, fractionation, digestion of the sample into peptides, depletion, enrichment, dialysis, desalting, alkylation and/or peptide reduction. However, these steps are optional. The selective detection of analyte ions may be conducted with tandem mass spectrometry (MS/MS). Tandem mass spectrometry is characterized by mass selection step (as used herein, the term "mass selection" denotes isolation of ions having a specified m/z or narrow range of m/z's), followed by fragmentation of the selected ions and mass analysis of the resultant product (fragment) ions.

The skilled person is aware how quantify the level of a marker in the sample by mass spectrometric methods. For example, relative quantification "rSRM" or absolute quantification can be employed as described above.

Moreover, the levels (including reference levels) can be determined by mass spectrometric based methods, such as methods determining the relative quantification or determining the absolute quantification of the protein or fragment thereof of interest.

Relative quantification "rSRM" may be achieved by:
1. Determining increased or decreased presence of the target protein by comparing the SRM (Selected reaction monitoring) signature peak area from a given target fragment peptide detected in the sample to the same SRM signature peak area of the target fragment peptide in at least a second, third, fourth or more biological samples.
2. Determining increased or decreased presence of target protein by comparing the SRM signature peak area from a given target peptide detected in the sample to SRM signature peak areas developed from fragment peptides from other proteins, in other samples derived from different and separate biological sources, where the SRM signature peak area comparison between the two samples for a peptide fragment are normalized for e.g. to amount of protein analyzed in each sample.
3. Determining increased or decreased presence of the target protein by comparing the SRM signature peak area for a given target peptide to the SRM signature peak areas from other fragment peptides derived from different proteins within the same biological sample in order to normalize changing levels of histones protein to levels of other proteins that do not change their levels of expression under various cellular conditions.
4. These assays can be applied to both unmodified fragment peptides and to modified fragment peptides of the target proteins, where the modifications include, but are not limited to phosphorylation and/or glycosylation, acetylation, methylation (mono, di, tri), citrullination, ubiquitinylation and where the relative levels of modified peptides are determined in the same manner as determining relative amounts of unmodified peptides.

Absolute quantification of a given peptide may be achieved by:
1. Comparing the SRM/MRM signature peak area for a given fragment peptide from the target proteins in an individual biological sample to the SRM/MRM signature peak area of an internal fragment peptide standard spiked into the protein lysate from the biological sample. The internal standard may be a labeled synthetic version of the fragment peptide from the target protein that is being interrogated or the labeled recombinant protein. This standard is spiked into a sample in known amounts before (mandatory for the recombinant protein) or after digestion, and the SRM/MRM signature peak area can be determined for both the internal fragment peptide standard and the native fragment peptide in the biological sample separately, followed by comparison of both peak areas. This can be applied to unmodified fragment peptides and modified fragment peptides, where the modifications include but are not limited to phosphorylation and/or glycosylation, acetylation, methylation (e.g. mono-, di-, or tri-methylation), citrullination, ubiquitinylation, and where the absolute levels of modified peptides can be determined in the same manner as determining absolute levels of unmodified peptides.
2. Peptides can also be quantified using external calibration curves. The normal curve approach uses a constant amount of a heavy peptide as an internal standard and a varying amount of light synthetic peptide spiked into the sample. A representative matrix similar to that of the test samples needs to be used to construct standard curves to account for a matrix effect. Besides, reverse curve method circumvents the issue of endogenous analyte in the matrix, where a constant amount of light peptide is spiked on top of the endogenous analyte to create an internal standard and varying amounts of heavy peptide are spiked to create a set of concentration standards. Test samples to be compared with either the normal or reverse curves are spiked with the same amount of standard peptide as the internal standard spiked into the matrix used to create the calibration curve.

The invention further relates to kits, the use of the kits and methods wherein such kits are used. The invention relates to kits for carrying out the herein above and below provided methods. The herein provided definitions, e.g. provided in relation to the methods, also apply to the kits of the invention. In particular, the invention relates to kits for therapy monitoring, comprising the prognosis, risk assessment or risk stratification of a subsequent adverse event in the health of a patient, wherein said kit comprises
- detection reagents for determining the level proADM or fragment(s) thereof, and optionally additionally for determining the level of PCT, lactate and/or C-reactive protein or fragment(s) thereof, in a sample from a subject, and - detection reagents for determining said level of ADM in said sample of said subject, and
- reference data, such as a reference level, corresponding to high and/or low severity levels of ADM, wherein the low severity level is below 4 nmol/l, preferably below 3 nmol/l, more preferably below 2.7 nmol/l, and the high severity level is above 6.5 nmol/l, preferably above 6.95 nmol/l, more preferably above 10.9 nmol/l, and optionally PCT, lactate and/or C-reactive protein levels, wherein said reference data is preferably stored on a computer readable medium and/or employed in the form of computer executable code configured for comparing the determined levels of proADM or fragment(s) thereof, and optionally additionally the determined levels of PCT, lactate and/or C-reactive protein or fragment(s) thereof, to said reference data.

As used herein, "reference data" comprise reference level(s) of ADM and optionally PCT, lactate, C-reactive protein and/or other suitable markers disclosed herein, such as for example markers of rhabdomyolysis. The levels of ADM and optionally other markers such as PCT, lactate and/or C-reactive protein in the sample of the subject can be compared to the reference levels comprised in the reference data of the kit. The reference levels are herein described above and are exemplified also in the appended examples. The reference data can also include a reference sample to which the level of ADM and optionally PCT, lactate and/or C-reactive protein is compared. The reference data can also include an instruction manual how to use the kits of the invention.

The kit may additionally comprise items useful for obtaining a sample, such as a blood sample, for example the kit may comprise a container, wherein said container comprises a device for attachment of said container to a cannula or syringe, is a syringe suitable for blood isolation, exhibits an internal pressure less than atmospheric pressure, such as is suitable for drawing a pre-determined volume of sample into said container, and/or comprises additionally detergents, chaotropic salts, ribonuclease inhibitors, chelating agents, such as guanidinium isothiocyanate, guanidinium hydrochloride, sodium dodecylsulfate, polyoxyethylene sorbitan monolaurate, RNAse inhibitor proteins, and mixtures thereof, and/or A filter system containing nitro-cellulose, silica matrix, ferromagnetic spheres, a cup retrieve spill over, trehalose, fructose, lactose, mannose, poly-ethylen-glycol, glycerol, EDTA, TRIS, limonene, xylene, benzoyl, phenol, mineral oil, anilin, pyrol, citrate, and mixtures thereof.

As used herein, the "detection reagent" or the like are reagents that are suitable to determine the herein described marker(s), e.g. of ADM, PCT, lactate and/or C-reactive protein. Such exemplary detection reagents are, for example, ligands, e.g. antibodies or fragments thereof, which specifically bind to the peptide or epitopes of the herein described marker(s). Such ligands might be used in immunoassays as described above. Further reagents that are employed in the immunoassays to determine the level of the marker(s) may also be comprised in the kit and are herein considered as detection reagents. Detection reagents can also relate to reagents that are employed to detect the markers or fragments thereof by MS based methods. Such detection reagent can thus also be reagents, e.g. enzymes, chemicals, buffers, etc, that are used to prepare the sample for the MS analysis. A mass spectrometer can also be considered as a detection reagent. Detection reagents according to the invention can also be calibration solution(s), e.g. which can be employed to determine and compare the level of the marker(s).

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having an infection and "disease" populations, e.g. subjects having an infection. For any particular marker (like ADM), a distribution of marker levels for subjects with and without a disease/condition will likely overlap. Under such conditions, a test does not absolutely distinguish normal from disease with 100% accuracy, and the area of overlap might indicate where the test cannot distinguish normal from disease. A threshold is selected, below which the test is considered to be abnormal and above which the test is considered to be normal or below or above which the test indicates a specific condition, e.g. infection. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art; see, e.g., Hanley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

Accordingly, the invention comprises the administration of an antibiotic suitable for treatment on the basis of the information obtained by the method described herein.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of".

Thus, the terms "comprising"/"including"/"having" mean that any further component (or likewise features, integers, steps and the like) can/may be present. The term "consisting of" means that no further component (or likewise features, integers, steps and the like) is present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of" means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

### EXAMPLES

The present invention is further described by reference to the following non-limiting examples.

### Background:

80% of cases of sepsis start in the community, before hospitalization. Success of Sepsis treatment is time dependant. Early diagnosis of sepsis in the community, for example in primary treatment centers, such as by medical personnel who first meet patients with symptoms of infectious disease, i.e. GPs, should be a priority to diminish morbidity and mortality of this disease.

### Primary Objective of the Study described in the Examples:

To find the best biomarkers to identify patients with suspected (preferably respiratory) infections in the community (i.e. at a GP) who require transfer to a hospital, i.e. the Emergency Room (ER) at the hospital, and differentiating them from those who are not transferred to the hospital.

The predictive ability of biomarkers will be compared against that of qSOFA score and NEW score.

### Design:

This is a prospective observational study recruiting patients with respiratory infection in the community. Patient recruitment takes place at a Community Health Center. GPs recruit the patients either at the Community Health Center or at patients' home.

Sampie size: 220 patients with suspected respiratory infection are assessed.

Inclusion criteria: adult patients with signs of respiratory infection (cough, pharyngeal pain, increase in mucus production, dyspnoea ...) scoring at least 1 point in the NEWS score:
Respiration rate: 11 rpm - 21 rpm.
Oxygen saturation: 95.
Temperature: 36.0 - 38.1 °C
Systolic blood pressure: 110 mmHg - 220 mmHg
Heart rate: 50 - 91

### Criteria for deciding patient's transfer to the hospital:

The attending physician decides based on their own clinical criteria. The study does not interfere with normal SOPs of GPs attending patients with respiratory infection in the community. Differences in severity between patients transferred and not transferred to hospital are analyzed at the end of the study using the data collected in the normalized datasheets.

Exclusion criteria: not giving informed consent, patients younger than 18 yr old.

### Clinical information:

The most relevant clinical data is collected using a standardized data sheet. Data collected allows calculating quick SOFA score and NEW score.

Sampies collected: Whole blood is collected at the time of patient inclusion, using 2 Paxgene tubes (2.5 ml of blood each) and 1 EDTA tube (5 ml). Paxgene tubes are stored at -20°C for a maximum of 3 months, and are transferred to a -80°C freezer. Blood from EDTA tubes is immediately processed for procalcitonin and proadrenomedullin quantification using a point of care (POC) device. A nasopharyngeal swab is collected also in parallel and stored at -20°C for a maximum of 3 months, and is transferred to a 8oC freezer.

### Biomarkers profiled:

Blood in Paxgene tubes is employed for profiling gene expression using microarrays, next generation sequencing or quantitative PCR methods using real time, droplet digital PCR or similar methods. Blood in EDTA tubes is used for procalcitonin and proadrenomedullin quantification employing a POC device provided by Thermo Fischer Scientific (B.R.A.H.M.S, Hennigsdorf, Germany), in the first 12 hours following inclusion of the patient.

Nasopharyngeal swabs will be employed for viral diagnosis using molecular biology methods.

Statistical analysis: AUROC and multivariate regression analysis are performed to test the ability of biomarkers to differentiate between those patients being transferred to the hospital and those who were not transferred. In those patients needing of Hospitalization, the same approach is employed to predict hospitalization at the ward or the ICU, or mortality. Multivariate linear regression models are built to predict length of hospitalization.

### Results:

Patients with respiratory infections (as assessed by the GP) were enrolled at a primary care facility. These patients included those with viral and bacterial infections (or a combination of the two).

MR-proADM at a cut-off of 1.2 nmol/L was an independent parameter associated with a hospital admission to the ward after being referred to by a GP (actually being admitted to the hospital is the determining factor, as opposed to just being referred and then sent away again).

Elevated PCT levels could identify whether a patient had a bacterial or viral infection, but was relatively poor compared to proADM at determining whether the patient needed to be sent to hospital or not.

### Solution:

Measurement of MR-proADM at a GP's surgery or other primary health care contact points can identify patients who are likely to require hospitalisation. This can decrease waiting time at a GPs surgery and within the ED (where they would still have to wait to be seen, despite a referral).

Patients with an MR-proADM concentration <1.2 nmol/L would not be sent to the hospital, and would be given antibiotics to take at home depending on their PCT concentration, thus decreasing patient numbers attending the ED.

Thus, a combination of both biomarkers can ensure that the GP doesn't send "unnecessary" patients to the ED, whilst providing antibiotics to those who can be treated at home. End result - decreased ED admissions, which represent a long-sought after solution to managing patient treatment when the severity of the disease appears uncertain. To the knowledge of the inventor, there aren't any previous primary care MR-proADM studies suggesting these results (and certainly none investigating infections).

### Data:

Total: n= 66
Not deserving hospitalization at the ward: n= 49
Admitted to hospital ward: n= 17

### Multivariate regression analysis for assessing the risk of hospitalization at the ward:

| | **OR** | **Cl95%** | | **p** |
|---|---|---|---|---|
| Antibiotics at home | 1,936 | 0,154 | 24,394 | 0,609 |
| O2 at home | 5,153 | 0,276 | 96,219 | 0,272 |
| Anti-hypertensive drugs | 0,066 | 0,002 | 2,138 | 0,125 |
| Oral steroids | 3,011 | 0,116 | 78,009 | 0,507 |
| Inhaled Steroids | 1,078 | 0,059 | 19,583 | 0,959 |
| High Blood Pressure | 27,877 | 0,797 | 974,607 | 0,066 |
| Temperature | 0,476 | 0,167 | 1,361 | 0,166 |
| Respiratory Rate | 1,089 | 0,926 | 1,28 | 0,304 |
| O2 Saturation | 0,791 | 0,601 | 1,04 | 0,093 |
| **ProADM > 1.2** | **8,112** | **1,095** | **60,09** | **0,04** |

## Claims

1. Method for therapy guidance, stratification and/or control in a patient with symptoms of an infectious disease, comprising a prognosis of disease progression, the method comprising:
- providing a sample from said patient, and
- determining a level of proADM or fragment(s) thereof in said sample,
- wherein a low risk level of proADM level or fragment(s) thereof in said sample indicates that the patient is not at risk of a disease progression to a condition that requires hospitalization, wherein said low risk level is equal or below 1.2 nmol/l ± 20%, and/or
- wherein a high risk level of proADM level or fragment(s) thereof in said sample indicates that the patient is at risk of a disease progression to a condition that requires hospitalization, wherein said high risk level is above 1.2 nmol/l ± 20%.

2. Method according to claim 1, wherein the level of proADM or fragment(s) thereof is determined in a sample obtained by the first medical personnel to consult with the patient after occurrence of the symptoms of infectious disease.

3. Method according to any one of the preceding claims, wherein at the time of sample provision the patient shows mild symptoms of an infectious disease corresponding to a qSOFA score of 0 or 1.

4. Method according to any one of the preceding claims, wherein at the time of sample provision the patient shows no clinical symptoms for a blood stream infection, a sepsis, a severe sepsis and/or septic shock in the patient.

5. Method according to any one of the preceding claims, wherein the level of proADM or fragment(s) thereof in said sample in between 0.7 nm/L ± 20% and 1.2 nmol/L ± 20% indicates the presence of the disease and that the patient is not at risk of a disease progression to a condition that requires a hospitalization.

6. Method according to any one of the preceeding claims, wherein a high risk level of proADM or fragments thereof indicates that the patient is at risk of disease progression to a life-threatening condition within 48 hours, 24 hours, preferably 12 hours and wherein a low risk level of proADM indicates that the patient is not at risk of disease progression to a life-threating condition within 48 hours, 24 hours, preferably 12 hours.

7. Method according to any one of the preceding claims, wherein the level of proADM or fragment(s) thereof in said sample is indicative of the risk of a development of a blood stream infection, sepsis, severe sepsis and/or septic shock in the patient that requires hospitalization.

8. Method according to claim 1, wherein a high risk level of proADM thereof indicates that the patient is at risk to develop a blood stream infection, sepsis, severe sepsis and/or septic shock that requires hospitalization within 48 hours, 24 hours, preferably 12 hours and wherein a low risk level of proADM indicates that the patient is not at risk to develop blood stream infection, a sepsis, a severe sepsis and/or a septic shock within 48 hours, 24 hours, preferably 12 hours.

9. Method according to any one of the preceding claims, wherein the level of proADM or fragment(s) thereof in said sample is indicative of the patient requiring frequent monitoring and/or critical care treatment.

10. Method according to any one of the preceding claims, additionally comprising
- determining at least one clinical score, preferably the NEWS score, for the patient suspected of having an infectious disease,
- wherein the at least one clinical score and the level of proADM or fragment(s) thereof is indicative of the presence of the infectious disease and whether the patient is at risk of a disease progression to a condition that requires a hospitalization.

11. Method according to any one of the preceding claims, wherein the infectious disease is a respiratory infectious disease, urinary tract infectious disease, a skin infectious disease or an abdominal infectious disease.

12. Method according to any one of the preceding claims, wherein determining a level of proADM or fragment(s) thereof comprises determining a level of MR-proADM in the sample.

13. Method according to any one of the preceding claims, wherein the sample is selected from the group consisting of a blood sample, such as a whole blood sample, a serum sample or a plasma sample, and/or a urine sample.

14. Method according to any one of the preceding claims, wherein the sample is isolated from the patients within 24 hours, 6 hours, 2 hours, 1 hour, more preferably within 30 minutes or 15 minutes after the symptoms have been determined.

15. Method according to any one of the preceding claims, additionally comprising
a. determining a level of at least one additional biomarker or fragment(s) thereof in a sample from said patient, wherein the at least one additional biomarker preferably is PCT or fragment(s) thereof, and
b. wherein the level of the at least one additional biomarker is indicative of an initiation of treatment with an anti-infective agent, preferably an antibiotic or antimitotic agent.

16. Method according to the preceding claim, wherein at least one additional biomarker is PCT or fragment(s) thereof and a level of PCT or fragment(s) thereof above 0,25 ng/ml, preferably above 0.5 ng/ml is indicative of an initiation of a treatment with an anti-infective agent, preferably an antibiotic or antimitotic agent.

17. Kit for carrying out the method of any one of the preceding claims, comprising:
a. detection reagents for determining the level of proADM or fragment(s) thereof in a sample from a patient, and
b. reference data for the risk of a patient as to whether a disease progression to a condition that requires hospitalization will occur, in particular reference data for a risk threshold or cut-off value, wherein said reference data is preferably stored on a computer readable medium and/or employed in the form of computer executable code configured for comparing the determined levels of proADM or fragment(s) thereof with the risk threshold or cut-off value,
c. and optionally, detection reagents for determining the level of at least one additional biomarker or fragment(s) thereof, preferably PCT, in a sample from a patient, and reference data, such as reference levels for said at least one additional biomarker, preferably PCT, for a risk threshold or cut-off value, wherein said reference data is preferably stored on a computer readable medium and/or employed in the form of computer executable code configured for comparing the determined levels of the at least one additional biomarker or fragment(s) thereof with the threshold or cut-off value.
